# EUROPÄISCHE PATENTANMELDUNG

(11) **EP 2 525 099 A1**
(43) Veröffentlichungstag der Anmeldung: **21.11.2012**
(21) Anmeldenummer: 11075086.6
(22) Anmeldetag: 16.05.2011
(51) Int. Cl.: F04D 3/02, F04D 29/046, A61M 1/10, F04D 29/047

(54) **Rotationspumpe mit zwei Lagerelementen und einem Fliesskanal**

(71) Anmelder: Berlin Heart GmbH, 12247 Berlin (DE)
(72) Erfinder: Gollner, Manfred, 13086 Berlin (DE); Donath, Johannes, 10829 Berlin (DE); Lehmann, Ulrich, 13127 Berlin (DE)
(74) Vertreter: Pfenning, Meinig & Partner GbR

(57) **Zusammenfassung**

Die Erfindung bezieht sich auf eine Rotationspumpe für ein Fluid, insbesondere eine Blutpumpe, mit einem Rotor, der an wenigstens einer Lagerstelle in einem Stator drehbar gelagert ist. Dabei ist an wenigstens einem der Lagerelemente (21, 22) außer einem Gleitbereich (21', 21'', 21''') wenigstens ein Kanalbereich vorgesehen, in dem zwischen den Lagerelementen ein Fließkanal (50) für ein Kühl- und/oder Schmierfluid gebildet ist. Die Lagerelemente können auch so gestaltet sein, dass durch Rotation des Rotors im Lager das Fluid in einem Fließkanal gefördert wird.

## Beschreibung

Die Erfindung liegt auf dem Gebiet der Mechanik und befasst sich speziell mit Rotationspumpen für Fluide.

Derartige Pumpen können beispielsweise als Axialpumpen oder Radialpumpen ausgebildet sein und weisen einen antreibbaren Rotor mit entsprechend bewegbaren Förderelementen auf. Die Pumpe fördert ein Fluid von einer Einlassseite her zu einer Auslassseite und schafft durch den Pumpenantrieb ein Druckgefälle.

Der Rotor der Pumpe ist azimutal, radial und axial wirkenden Kräften durch das Fluid ausgesetzt und muss in einem Rotationslager gelagert werden. An ein solches Lager werden oft erhöhte Anforderungen dadurch gestellt, dass beispielsweise das zu fördernde Fluid mit dem Lager in Berührung kommt und dort beispielsweise unerwünschte Wirkungen wie Korrosion hervorrufen kann. Es kann jedoch auch vorgesehen sein, dass das zu fördernde Fluid als Schmier- und/oder Kühlmittel im Lager wirkt.

Auch an die Toleranz des Lagers werden je nach Einsatzfall unterschiedliche, oft sehr hohe Anforderungen gestellt. Insbesondere bei sehr schnell laufenden Pumpen müssen Reibung und Verschleiß minimiert sein.

Eine besondere Gruppe von Fluidpumpen stellen die medizinisch anwendbaren Pumpen dar, die zur Förderung von pharmakologisch wirksamen oder beispielsweise körpereigenen Flüssigkeiten dienen. Bei diesen muss außer den oben angeführten Anforderungen auch darauf geachtet werden, dass nicht durch Scherwirkungen im Lager das zu fördernde Fluid auf Zellebene geschädigt wird. In diesem Zusammenhang sind bereits magnetisch gelagerte Rotoren und auch hochpräzise gearbeitete Lager mit besonders harten und verschleißarmen Lagerflächen vorgeschlagen worden.

Der vorliegenden Erfindung liegt die Aufgabe zugrunde, eine Rotationspumpe zu schaffen, die in Bezug auf ihre Lagerung im Hinblick auf die oben angeführten Aspekte optimiert ist. Dabei soll das Lager möglichst einfach und kostengünstig herstellbar sein.

Die Aufgabe wird mit den Merkmalen der Erfindung gemäß Patentanspruch 1 gelöst.

Dazu ist bei einer erfindungsgemäßen Rotationspumpe wenigstens ein Rotationslager mit einem ersten, mit dem Rotor verbundenen Lagerelement vorgesehen, das in oder an einem zweiten Lagerelement drehbar gelagert ist. Das erste und das zweite Lagerelement weisen jeweils wenigstens einen Gleitbereich auf, der an dem jeweils anderen Lagerelement gleitend anliegt. Zudem ist an wenigstens einem der Lagerelemente benachbart zu einem Gleitbereich wenigstens ein Kanalbereich vorgesehen, der von dem jeweils anderen Lagerelement unter Bildung eines Lagerspaltes dauerhaft beabstandet ist, so dass in dem Kanalbereich zwischen den Lagerelementen ein Fließkanal für ein Fluid gebildet ist.

Über den genannten Fließkanal kann ein Fluid, das der Schmierung und/oder Kühlung des Lagers dient, frei fließen, so dass es besonders gut in die Gleitbereiche gelangen kann. Dadurch werden die Gleitbereiche in der Lauffläche des Lagers besonders effizient mit dem Fluid versorgt.

Vorteilhaft kann dabei vorgesehen sein, dass die Tiefe des Fließkanals senkrecht zur Lauffläche des Lagers gemessen wenigstens dem Doppelten, insbesondere dem Dreifachen, vorteilhaft wenigstens dem Fünffachen des Lagerspaltes in den Gleitbereichen entspricht. Der jeweilige Querschnitt des Fließkanals kann dabei so groß ausgestaltet sein, dass eine Förderung des Fluids durch den Fließkanal mittels eines Druckgefälles möglich ist. Es kann jedoch auch vorgesehen sein, dass die Abmessungen des Kanals so gewählt sind, dass das Fluid in dem Fließkanal auch schon durch Kapillarwirkung gefördert wird.

Eine weitere vorteilhafte Ausgestaltung sieht vor, dass der Fließkanal langgestreckt mit einem ersten und einem zweiten Ende ausgebildet ist, mit einer sich zwischen dem ersten Ende und dem zweiten Ende erstreckenden Länge, die einem Mehrfachen seiner Breite und der senkrecht zur Lauffläche des Lagers gemessenen Tiefe des Fließkanals entspricht, und dass wenigstens ein Ende, vorzugsweise beide Enden, des Fließkanals mit einem oder zwei bzw. mehreren verschiedenen Fluidreservoir(en) außerhalb des Lagers verbunden sind.

Insbesondere kann der Fließkanal an einem Ende mit dem Raum außerhalb des Lagers verbunden sein, in dem sich das durch die Rotationspumpe zu fördernde Fluid bewegt. Dieses Fluid kann dann über den Fließkanal effizient durch Lager befördert werden, um dort als Schmiermittel und/oder Kühlmittel zu wirken.

Auch das andere Ende des Fließkanals kann vorteilhaft mit einem Fluidreservoir verbunden sein, wobei es sich als nützlich auswirkt, wenn ein Druckunterschied zwischen den beiden Enden des Fließkanals herrscht, so dass über das Druckgefälle das Fluid durch den Fließkanal gefördert wird. Es wird üblicherweise über einen solchen zusätzlichen Kanal in der Rotationspumpe die eigentliche Förderleistung der Pumpe reduziert, jedoch ist die Menge des durch den Fließkanal beförderten Fluids so gering, dass der Leistungsverlust der Pumpe nicht ins Gewicht fällt.

Eine weitere vorteilhafte Ausgestaltung der Erfindung sieht vor, dass der Lagerspalt des Rotationslagers in den Gleitbereichen kleiner ist als der Durchmesser eines Blutkörpers. Diese Ausgestaltung ist dann vorteilhaft, wenn die Rotationspumpe als Blutpumpe eingesetzt wird, beispielsweise auch als implantierbare oder ambulant einsetzbare Herzunterstützungspumpe oder Herzpumpe. Die entsprechende Gestaltung des Lagerspaltes bewirkt, dass keine Blutkörperchen, seien es rote oder weiße Blutkörperchen, oder andere Partikel des Blutes in die Gleitbereiche zwischen den Lagerelementen gelangen können, so dass dort schädigende Wirkungen auf das Blut, die durch Scherbelastungen auftreten könnten, vermieden werden. Die größeren Partikel im Blut können sich ausschließlich über den Fließkanal bewegen, während nur das Blutplasma in die Gleitbereiche zwischen den Lagerelementen eindringen und dort als Schmier- und/oder Kühlmittel wirken kann.

Vorteilhaft kann in dem Lager der Rotationspumpe auch der Fließkanal derart geformt sein, dass in ihm durch die Rotationsbewegung eines Lagerelementes das Fluid gefördert wird. Dies kann beispielsweise dadurch realisiert werden, dass ein Fließkanal wenigstens teilweise spiralförmig oder gewindeartig an einer zylindrischen, konischen oder balligen Oberfläche, beispielsweise auch einer Stirnfläche eines der Lagerelemente verläuft. Auch eine entsprechende Anordnung an einer Stirnseite des Rotationskörpers ist möglich.

Die Erfindung kann außerdem vorteilhaft dadurch ausgestaltet werden, dass in einem rotierenden und/oder einem nicht rotierenden Lagerelement wenigstens ein Förderkanal angeordnet ist, dessen Länge wenigstens zweimal, vorteilhaft wenigstens fünfmal so groß ist wie seine Maximalabmessung im Querschnitt und der sich der Länge nach zwischen einem ersten Ende und einem zweiten Ende erstreckt, wobei eines der Enden im Bereich zwischen den Lagerelementen liegt und einen anderen Abstand von der Rotationsachse aufweist als das andere Ende. Ein derartiger Förderkanal kann insbesondere zusätzlich zu einem Fließkanal und/oder als dessen Verlängerung vorgesehen werden, um den Fließkanal entweder radial mit einem Fluidraum zu verbinden und Druckunterschiede zur Förderung des Fluids zu nutzen oder auch durch Zentrifugalkräfte in dem Förderkanal selbst das Fluid anzutreiben.

Der Förderkanal kann in einigen Fällen auch mit dem Fließkanal zusammenfallen.

Vorteilhaft kann auch vorgesehen sein, dass das eine Ende des Förderkanals im Bereich zwischen den Lagerelementen am Lagerspalt angeordnet ist, während das andere Ende eine Mündung des Förderkanals an der Außenseite des Lagers darstellt. Das Fluid kann dann durch Zentrifugalkraft durch den Förderkanal radial nach außen gefördert und dort ausgestoßen werden, während neues Fluid über einen Fließkanal nachgezogen wird.

Im einfachsten Fall kann wenigstens ein Förderkanal als Radialbohrung in dem rotierenden und/oder dem ruhenden Lagerelement ausgebildet sein.

Es kann auch vorgesehen sein, dass ein Förderkanal an der Stirnseite eines der Lagerelemente ausgebildet und insbesondere zur Stirnseite hin offen ist.

Der Förderkanal kann dann in Bezug auf eine axiale Lagerung, wenn diese im Bereich der Stirnseite der Lagerelemente beabsichtigt ist, auch einen Fließkanal bilden.

Es kann auch vorteilhaft bei der erfindungsgemäßen Rotationspumpe vorgesehen sein, dass das Lager in Bezug auf die radiale und/oder die axiale Lagerung der Lagerelemente eine magnetische Lagervorrichtung aufweist. Insbesondere dann, wenn hauptsächlich eine Radiallagerung durch die genannten mechanischen Lagerelemente mit einem erfindungsgemäßen Fließkanal und einer Fluidschmierung vorgesehen ist, kann parallel dazu ein magnetisches Radiallager vorgesehen sein, das die Radialkräfte auf das erste Radiallager verringert und einen Teil der Lagerkräfte aufnimmt. Stattdessen oder zusätzlich kann zudem auch eine axiale Magnetlagerung für den Rotor der Rotationspumpe vorgesehen sein. Diese ist üblicherweise durch Magnetringe im Rotor und entsprechende im Stator vorgesehene Magnetringe verwirklicht. Die Magnetlager können wunschgemäß steifer oder weniger steif ausgebildet sein, je nachdem, wie weit die Gleitlager entlastet werden sollen. Es können auch, um den Justieraufwand zu reduzieren, geregelte Magnetlager eingesetzt werden.

Im Folgenden wird die Erfindung anhand eines Ausführungsbeispiels in einer Zeichnung gezeigt und nachfolgend beschrieben. Dabei zeigt
- Fig. 1: schematisch eine Rotationspumpe in einem Längsschnitt mit zwei Lagerstellen,
- Fig. 2: einen vergrößerten Ausschnitt einer Lager-stelle aus Fig. 1,
- Fig. 3: einen Längsschnitt einer anderen Rotations-pumpe mit Lagern, die sich von denen aus Fig. 1 unterscheiden,
- Fig. 4: ein vergrößertes Detail aus Fig. 3,
- Fig. 5: einen Längsschnitt und einen Querschnitt durch eine Lagerbuchse,
- Fig. 6: eine dreidimensionale Ansicht eines Wellen-endes,
- Fig. 7: einen Querschnitt durch eine weitere Lager-buchse mit einem entsprechenden Längs-schnitt,
- Fig. 8: eine weitere dreidimensionale Ansicht eines Wellenendes mit Abweichungen von der Kreis-Zylinderform,
- Fig. 9: einen Querschnitt durch eine Lagerbuchse mit einer in dieser geführten Welle,
- Fig. 10: einen Längsschnitt durch eine Rotations-pumpe mit einem Lager, das Radialbohrungen aufweist,
- Fig. 11: das Ende des Rotationskörpers, das an seiner Stirnseite eine Spiralnut aufweist,
- Fig. 12: einen Längsschnitt durch eine Rotations-pumpe mit einem zusätzlich zu den erfindungsgemäßen Lagern vorgesehenen magnetischen Axiallager,
- Fig. 13: einen weiteren Längsschnitt durch eine Rotationspumpe, ebenfalls mit zusätzlichen magnetischen Axiallagern, wobei ein Magnet-ring jeweils außen auf der Pumpe aufgesetzt und fixiert ist, und
- Fig. 14: einen Wellenbolzen in dreidimensionaler Darstellung mit einer umlaufenden Nut.

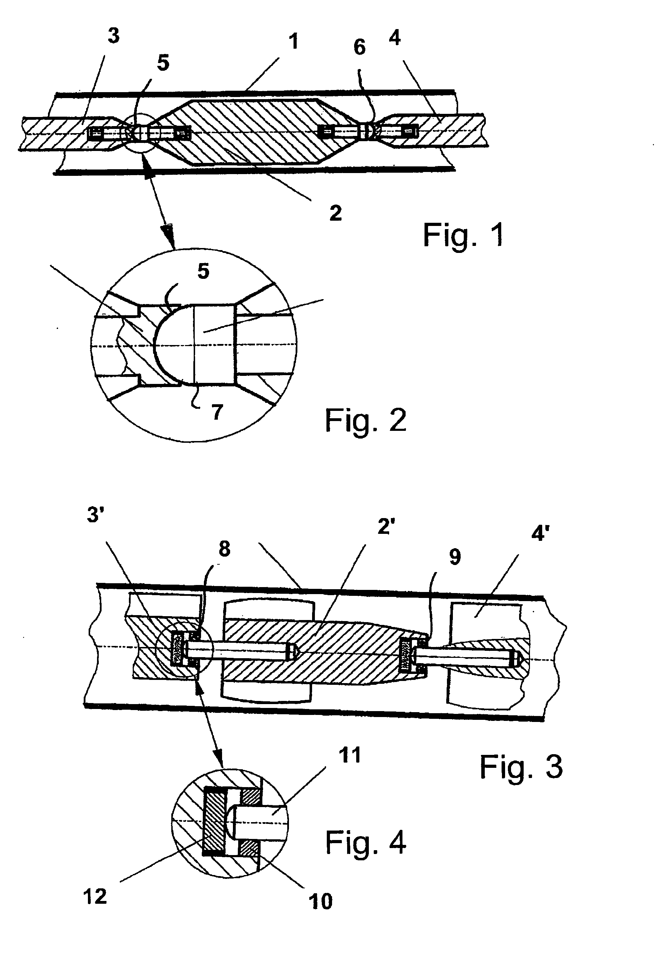

**Figur 1** zeigt in einem Längsschnitt ein Pumpengehäuse 1 mit einem darin angeordneten Rotor 2, der in einem ersten Gegenlager 3 und in einem zweiten Gegenlager 4 jeweils in kalottenförmigen Lagerbuchsen 5, 6 gelagert ist. Jede der Lagerbuchsen weist demnach, wie in **Figur 2** vergrößert gezeigt ist, eine kalottenförmige Lagerbuchse 5 auf, in die ein kalottenförmiger Bolzen 7 eingeführt ist, der in der Lagerbuchse rotiert.

Ein weiteres Lagerprinzip ist in **Figur 3** gezeigt, in der ein Rotor 2' in einem ersten Lager 3' und einem zweiten Lager 4' jeweils in Rubinbuchsen 8, 9 gelagert ist. Eine derartige Rubinbuchse ist in **Figur 4** vergrößert gezeigt. Es ist dabei ein Rubinring 10 dargestellt, der eine Öffnung für das Wellenende 11 aufweist, sowie eine Anlaufscheibe 12, die die Axialkräfte der Welle aufnimmt.

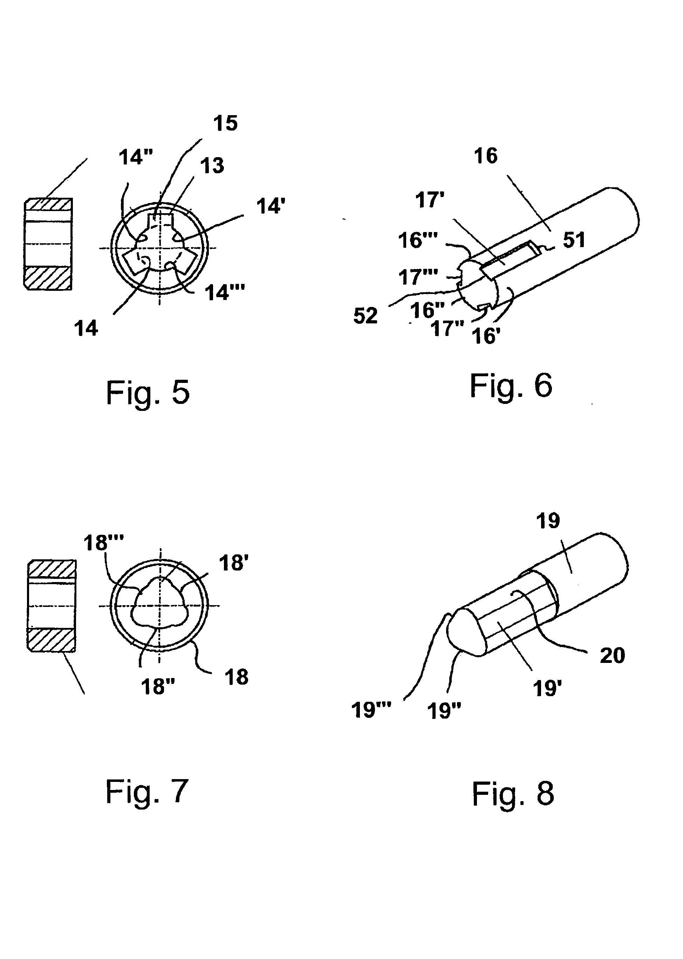

**Figur 5** zeigt in einem Querschnitt und in einem Längsschnitt eine Lagerbuchse 13, die eine zylindrische Lauffläche 14 aufweist, mit drei Gleitbereichen 14', 14'', 14'''. In diesem Bereich gleitet eine zylindrische Welle, die in die Buchse 13 eingeführt wird, an den Gleitbereichen. Zwischen den Gleitbereichen 14', 14'', 14''' ist die zylindrische Lauffläche gestrichelt dargestellt, und dort sind Kanalbereiche angeordnet, in denen die Buchse radial erweitert ist. Dort bilden sich Fließkanäle 15, in denen die zu fördernde Flüssigkeit in Axialrichtung an der nicht dargestellten Welle zwischen dieser und der Buchse 13 entlangfließen kann.

In **Figur 6** ist in dreidimensionaler Darstellung ein Wellenende 16 gezeigt, das umlaufend drei teilzylindrische Gleitbereiche 16', 16'', 16''' aufweist, die jeweils durch Kanalbereiche 17', 17'', 17''' voneinander getrennt sind.

Wenn die dargestellte Welle 16 in eine vollständig zylindrische Lagerbuchse eingeführt wird, so ergeben sich in den Kanalbereichen 17', 17'', 17''' zwischen der zylindrischen Buchseninnenfläche und der Welle 16 Fließkanäle, in denen das zu fördernde Fluid sich bewegen und ausbreiten kann. Die Kanalbereiche können am Umfang der Welle auch wendelförmig umlaufen. Auch an der Stirnseite der Welle können entsprechende Nuten vorgesehen sein.

**Figur 7** zeigt in einem Querschnitt eine weitere Lagerbuchse 18, die eine Buchsenöffnung mit teilzylindrischen Gleitflächen 18', 18'', 18''' aufweist, wobei jeweils zwischen den Gleitbereichen rinnenförmige Erweiterungen liegen, die entsprechende Kanalbereiche zwischen einer zylindrischen, gegebenenfalls in die Buchse eingeführten Welle und der Buchse bilden.

In **Figur 8** ist in dreidimensionaler Darstellung ein Wellenende 19 gezeigt, das von einer axialsymmetrischen Gestaltung dadurch abweicht, dass am Umfang des Wellenendes Abflachungen 19', 19'', 19''' gebildet sind, die zwischen teilzylindrischen Gleitbereichen 20 jeweils Kanalbereiche bilden, die in Zusammenwirkung mit einer zylindrischen Lagerbuchse Fließkanäle zum Transport eines Fluids ausbilden. In den Gleitbereichen kann die Breite des Lagespalts beispielsweise auf maximal 0,005 mm (Durchmesserunterschied zwischen Buchse und Welle, bei einseitig anliegender Welle entspricht dies dem Lagerspalt) begrenzt sein.

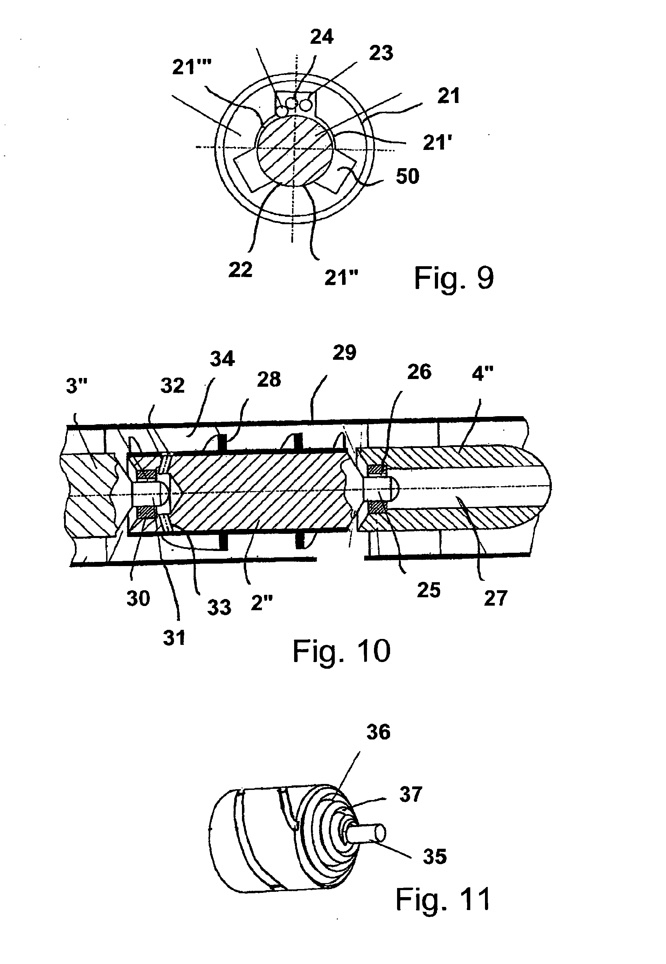

**Figur 9** zeigt im Querschnitt das Zusammenspiel einer Lagerbuchse 21 mit einer vollzylindrischen Welle 22. Die Lagerbuchse 21 weist Gleitbereiche 21', 21'', 21''' auf, in denen die Welle so genau an die Maße der Buchse angepasst ist, dass der Lagerspalt dort schmaler ist als der Durchmesser von Partikeln 23, 24, die in dem zu fördernden Fluid schwimmen, beispielsweise Blutpartikeln. Diese sammeln sich dementsprechend ausschließlich in den Fließkanälen, die durch Erweiterungen der Buchse 21 gebildet sind. Die Formen der Fließkanäle können, wie gezeigt, im Querschnitt rechteckig oder halbrund, jedoch auch dreieckig oder fünf- oder sechseckig gestaltet sein. Die Tiefe der Kanäle, gemessen von der Gegenfläche des jeweils anderen Lagerelementes bis zum Grund des Kanals, kann wenigstens 0,5 mm betragen.

Dadurch lässt sich bei Einsatz der Pumpe als Rotationspumpe für Blutförderung sicherstellen, dass in dem Lagerspalt ausschließlich Blutplasma gefördert wird und dass Blutkörperchen dort nicht durch Scher- oder Druckbeanspruchung geschädigt werden können. Das Blutplasma übernimmt somit die Schmier- und Kühlfunktionen. Blutplasma und Blutkörper zusammen können ausschließlich durch die Fließkanäle an den Erweiterungen der Buchse fließen.

**Figur 10** zeigt in einem Längsschnitt eine Rotationspumpe mit einem Rotor 2'', der an zwei Statoren 3'', 4'' gelagert ist. Dabei weist der Rotor an einem Ende einen Wellenzapfen 25 auf, der in einer ortsfesten Buchse 26 des Stators 4'' gelagert ist. Die Buchse 26 kann gemäß den in den Figuren 5, 7 und 9 gezeigten Varianten ausgeführt sein.

Der Stator 4'' ist hohl ausgebildet, mit einem zentrischen Kanal 27, durch den das Fluid, das die Buchse 26 durchströmt, einströmen kann.

Der Rotor 2'' weist an seiner Außenseite Förderelemente in Form von Schaufeln 28 auf, die bei Rotation beispielsweise im Drehzahlbereich zwischen 10.000 und 20.000 Umdrehungen pro Minute das in dem Gehäuse 29 befindliche Fluid fördern.

Der Rotor 2'' weist an seinem dem Wellenbolzen 25 gegenüberliegenden Ende eine Lagerbuchse als Lagerelement 30 auf, die ebenfalls gemäß den Formen der Erfindung, beispielsweise nach Figuren 5, 7 oder 9, ausgebildet sein kann. Der Bolzen 31, der das andere Lagerelement bildet, ist feststehend ausgebildet und mit dem Stator 3'' verbunden.

Um ein Abströmen des Fluids, das durch die Lagerbuchse 30 und zwischen dieser und dem Bolzen 31 hindurchströmt, zu ermöglichen, sind Radialbohrungen 32, 33 vorgesehen, die den Raum hinter der Buchse 30 mit dem Förderraum 34 verbinden, in dem sich die Förderelemente 28 bewegen.

Durch eine schnelle Rotation des Rotors 2'' tritt zudem eine Beschleunigung des Fluids in den Radialbohrungen 32, 33 auf, so dass hier eine Zentrifugalpumpe für das Fluid im Buchsenbereich gebildet ist.

**Figur 11** zeigt die Stirnfläche des mit einem Lagerzapfen versehenen Rotationskörpers, dessen Beschaufelung nicht dargestellt ist. In der Stirnfläche 36 ist eine Spiralnut 37 vorgesehen, und in dieser wird bei Rotation je nach Drehrichtung das Fluid radial von außen nach innen oder von innen nach außen durch die Buchse 26 gefördert. Vorteilhaft ist dabei die Förderung von innen nach außen entsprechend Fig. 10. In diesem Fall kann zwischen dem Wellenbolzen und einer Lagerbuchse das Fluid an der Mantelfläche 35 des Bolzens entlangströmen und zur Stirnseite 36 strömen, wo es radial nach außen in den Strömungsraum 34 geführt und von dort weitergeleitet wird. Die Spiralnut 37 erzeugt dabei eine pumpende Wirkung.

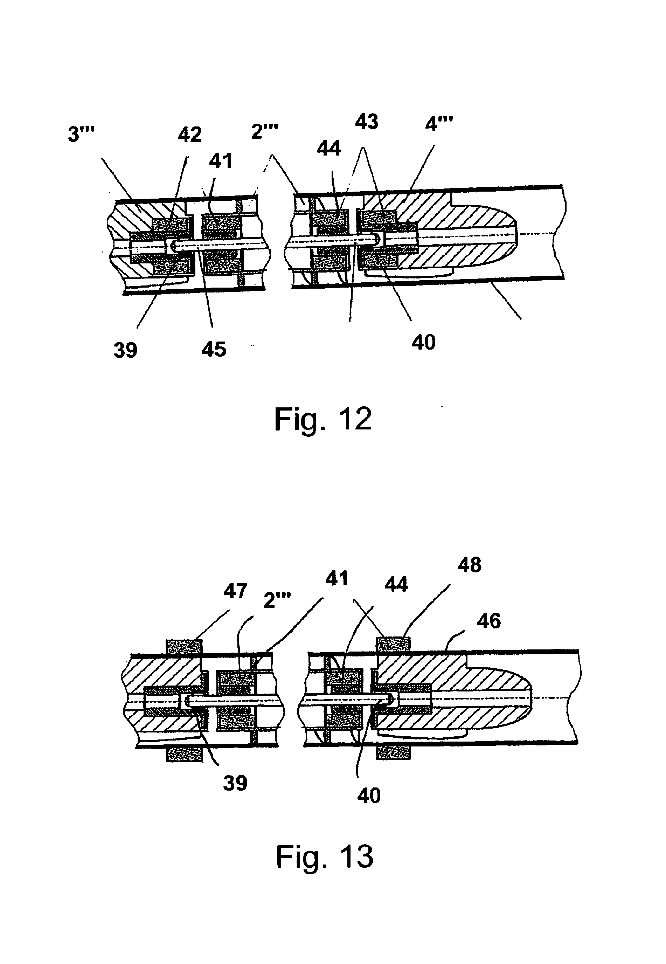

In **Figur 12** ist im Längsschnitt eine Rotationspumpe schematisch dargestellt, wobei der Rotor durch axial wirkende magnetische Kräfte passiver Magnetlager gelagert (eingeklemmt) ist. Die Darstellung ist aus Maßstabsgründen in Axialrichtung des Rotors unterbrochen.

In der Darstellung der Fig. 12 sind für die Welle 45 des Rotors keine Anlaufplatten vorgesehen, so dass die beschriebenen magnetischen Axiallager die wirkenden Axialkräfte voll aufnehmen. Es kann jedoch auch zusätzlich an einem oder beiden Enden des Rotors jeweils in dem Stator eine Anlaufplatte vorgesehen sein, die beim Auftreten unvorhergesehener oder außergewöhnlicher Axialkräfte die Axialbewegung des Rotors begrenzt und die Kräfte aufnimmt. Zudem können auch an den Lagerstellen 39, 40 radial wirkende Magnetlager als Unterstützung vorgesehen sein.

**Figur 13** zeigt in Abweichung von Fig. 12 einen Rotor 2''', der über zwei Axialmagnetlager gelagert ist, wobei der Rotor 2''' an seinen Enden jeweils einen Magnetring 41, 44 aufweist. Im Gegensatz zu der Ausführungsform der Fig. 12 ist der jeweilige magnetische Gegenring außerhalb des Gehäuses 46 angeordnet. Dort liegen die ortsfesten Magnetringe 47, 48, um den Rotor 2''' axial zu lagern. Zudem kann durch den im Verhältnis zu den Magnetringen im Rotor größeren Durchmesser der Magnetringe 47, 48 außerhalb des Gehäuses teilweise eine Radialstabilisierung erreicht werden, die die Wirkung der Radiallagerstellen 39, 40 unterstützt.

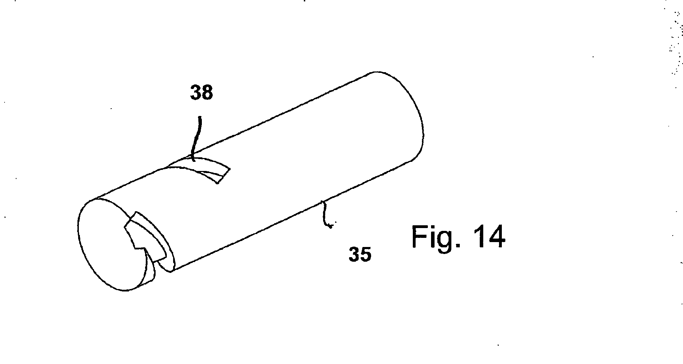

**Figur 14** zeigt in dreidimensionaler Darstellung das Ende eines Wellenbolzens, der an seiner Mantelfläche 35 eine wendelförmige Nut 38 (schematisch dargestellt) aufweist, die bei Rotation eine fluidfördernde Wirkung erzeugt.

## Patentansprüche

1. Rotationspumpe für ein Fluid mit einem Rotor (2, 2', 2'', 2'''), der um eine Rotationsachse rotiert, und mit wenigstens einem Rotationslager, in dem ein erstes, mit dem Rotor verbundenes Lagerelement in oder an einem zweiten Lagerelement (13, 16, 18, 19, 21, 22, 25, 26, 30, 31) drehbar gelagert ist, wobei das erste und das zweite Lagerelement jeweils wenigstens einen Gleitbereich (14', 14'', 14''', 18', 18'', 18''', 21' , 21'', 21''') aufweisen, der an dem jeweils anderen Lagerelement gleitend anliegt,
**dadurch gekennzeichnet,**
**dass** an wenigstens einem der Lagerelemente benachbart zu einem Gleitbereich wenigstens ein Kanalbereich (15, 17', 17'', 17''', 19', 19'', 19''') vorgesehen ist, der von dem jeweils anderen Lagerelement dauerhaft unter Bildung eines erweiterten Lagerspaltes in Radialrichtung bezüglich der Rotationsachse beabstandet ist, so dass in dem Kanalbereich zwischen den Lagerelementen ein Fließkanal (50) für ein Fluid gebildet ist.

2. Rotationspumpe nach Anspruch 1, **dadurch gekennzeichnet, dass** die Breite des Lagerspaltes im Bereich des Fließkanals (50) wenigstens dem Doppelten, insbesondere dem Dreifachen, vorteilhaft wenigstens dem Fünffachen des Lagerspaltes in den Gleitbereichen (14', 14'', 14''', 18', 18'', 18''', 21', 21'', 21''') entspricht.

3. Rotationspumpe nach Anspruch 1 oder 2, **dadurch gekennzeichnet, dass** der Fließkanal (17') langgestreckt mit einem ersten und einem zweiten Ende (51, 52) ausgebildet ist, mit einer sich zwischen dem ersten Ende (51) und dem zweiten Ende (52) erstreckenden Länge, die einem Mehrfachen seiner Breite und der senkrecht zur Lauffläche des Lagers gemessenen Tiefe des Fließkanals entspricht, und dass beide Enden des Fließkanals mit einem oder zwei Fluidreservoir(en) außerhalb des Lagers verbunden sind.

4. Rotationspumpe nach Anspruch 1 oder einem der folgenden, **dadurch gekennzeichnet, dass** der Lagerspalt des Rotationslagers in den Gleitbereichen (14', 14'', 14''', 18', 18'', 18''', 21', 21'', 21''') kleiner ist als der Durchmesser eines Blutkörpers (23, 24).

5. Rotationspumpe nach Anspruch 1 oder einem der folgenden, **dadurch gekennzeichnet, dass** der Fließkanal (37, 38) derart geformt ist, dass in ihm durch die Rotationsbewegung eines Lagerelementes das Fluid gefördert wird.

6. Rotationspumpe nach Anspruch 1 oder einem der folgenden, **dadurch gekennzeichnet, dass** in einem rotierenden und/oder einem nicht rotierenden Lagerelement wenigstens ein Förderkanal (32, 33, 37) angeordnet ist, dessen Länge wenigstens zweimal, vorteilhaft wenigstens fünfmal so groß ist wie seine Maximalabmessung im Querschnitt und der sich der Länge nach zwischen einem ersten Ende und einem zweiten Ende erstreckt, wobei eines der Enden im Bereich zwischen den Lagerelementen liegt und einen anderen Abstand von der Rotationsachse aufweist als das andere Ende.

7. Rotationspumpe nach Anspruch 6, **dadurch gekennzeichnet, dass** das andere Ende eine Mündung des Förderkanals (32, 33) an der Außenseite des Lagers bildet.

8. Rotationspumpe nach Anspruch 6 oder 7, **dadurch gekennzeichnet, dass** wenigstens ein Förderkanal (32, 33) als Radialbohrung in oder an dem rotierenden oder dem ruhenden Lagerelement ausgebildet ist.

9. Rotationspumpe nach Anspruch 6 oder 7, **dadurch gekennzeichnet, dass** ein Förderkanal (37) an der Stirnseite eines der Lagerelemente ausgebildet und insbesondere zur Stirnseite hin offen ist.

10. Rotationspumpe nach Anspruch 1 oder einem der folgenden, **dadurch gekennzeichnet, dass** das Lager in Bezug auf die radiale und/oder die axiale Lagerung der Lagerelemente eine magnetische Lagervorrichtung (41, 42, 43, 44, 47, 48) aufweist.
